(19) Europäisches Patentamt
European Patent Office
Office européen des brevets

(11) EP 1 926 391 B1

(12) FASCICULE DE BREVET EUROPEEN

(45) Date de publication et mention de la délivrance du brevet:
**12.08.2009 Bulletin 2009/33**

(21) Numéro de dépôt: **06808106.6**

(22) Date de dépôt: **11.09.2006**

(51) Int Cl.:
***A23K 1/16*** *(2006.01)* ***A23K 1/18*** *(2006.01)*
***A61K 31/198*** *(2006.01)* ***A61K 36/00*** *(2006.01)*

(86) Numéro de dépôt international:
**PCT/FR2006/002080**

(87) Numéro de publication internationale:
**WO 2007/031632 (22.03.2007 Gazette 2007/12)**

(54) **COMBINAISON DE MÉTHIONINE BIODISPONIBLE AVEC AU MOINS UNE HUILE ESSENTIELLE**

KOMBINATION AUS BIOVERFÜGBAREM METHIONIN UND WENIGSTENS EINEM ÄTHERISCHEN ÖL

COMBINATION OF BIOAVAILABLE METHIONINE WITH AT LEAST ONE ESSENTIAL OIL

(84) Etats contractants désignés:
**AT BE BG CH CY CZ DE DK EE ES FI FR GB GR HU IE IS IT LI LT LU LV MC NL PL PT RO SE SI SK TR**

(30) Priorité: **12.09.2005 FR 0509273**

(43) Date de publication de la demande:
**04.06.2008 Bulletin 2008/23**

(73) Titulaire: **Adisseo Ireland Ltd**
**Dublin 1 (IE)**

(72) Inventeurs:
- **MERCIER, Yves**
**F-63000 Clermont Ferrand (FR)**
- **GERAERT, Pierre-André**
**F-37210 Rochecorbon (FR)**

(74) Mandataire: **Jouannic, Nathalie et al**
**Cabinet Germain & Maureau**
**BP 6153**
**69466 Lyon Cedex 06 (FR)**

(56) Documents cités:
**EP-A- 1 314 358**
**EP-A- 1 314 359**
**WO-A-03/056935**
**US-A- 4 166 867**
**US-A1- 2002 146 399**
**US-A1- 2004 241 194**
**US-B1- 6 322 825**
**US-B1- 6 451 861**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 1 926 391 B1

## Description

La présente invention concerne une composition alimentaire, destinée à complémenter l'alimentation des animaux d'élevage en méthionine.

La microflore intestinale des animaux est composée de diverses communautés complexes, regroupant des bactéries, des levures et des champignons. Cette microflore a un impact important sur la santé et l'efficacité alimentaire des animaux.

On retrouve généralement deux types de bactéries : d'une part les bactéries pathogènes (par exemple, *Salmonella, Campylobacter jejuni* ou *Clostridium perfringens*), et d'autre part, les bactéries qui ne sont pas considérées comme pathogènes (par exemple, *Lactobacillus* ou *Propionibacterium*). Le bilan global des effets de ces bactéries sur les performances des animaux d'élevage est négatif.

Depuis de nombreuses années, les sociétés impliquées dans l'élevage des animaux cherchent à orienter la flore bactérienne intestinale afin d'améliorer les performances des animaux.

Ainsi le document US 6,322,825 décrit l'utilisation d'huiles essentielles dans la préparation de compositions pharmaceutiques pour remplacer les compositions comprenant notamment des sulfonamides de la cortisone ou des antibiotiques.

Le document US 4,166,867 décrit quant à lui la préparation d'aliment pour chevaux qui soient plus savoureux, notamment grâce à l'utilisation de l'huile essentielle de citron comme enrobant, l'huile représentant moins de 0.01% en poids du produit final.

La demande de brevet WO 03/056935 décrit un complément alimentaire utilisable dans la formulation d'aliments pour animaux ruminants lequel comprend au moins une huile essentielle et au moins une saponine ledit complément agissant dans le rumen de manière à maintenir un taux optimal de protéines ruminales.

Il est notamment courant de complémenter l'alimentation des animaux avec des antibiotiques facteurs de croissance, ce qui permet de diminuer la charge bactérienne globale du tube digestif et d'améliorer les performances de croissance ou de production. Cependant, l'utilisation des antibiotiques en tant que facteurs de croissance est interdite en Europe depuis le 1er janvier 2006.

Un besoin se fait donc sentir pour des produits de remplacement.

Un objet de la présente invention est de proposer une composition destinée à l'alimentation des animaux d'élevage ayant un effet bactéricide sur la flore intestinale des animaux d'élevage.

Un autre objet de la présente invention est de proposer une composition destinée à l'alimentation des animaux d'élevage ayant un effet bénéfique sur les performances des animaux.

Un autre objet de la présente invention est de proposer une composition alimentaire qui ne comprend pas d'antibiotiques, tout en ayant les effets bénéfiques que présentent généralement les antibiotiques sur la croissance des animaux.

Un autre objet encore de la présente invention est de proposer une composition alimentaire ayant une action sur la flore intestinale des animaux d'élevage.

La présente invention concerne donc une composition synergique alimentaire de méthionine biodisponible destinée à l'alimentation des animaux d'élevage, comprenant :

a) un composé de méthionine biodisponible, et
b) au moins 5% d'au moins une huile essentielle.

Les inventeurs ont constaté que la composition de la présente invention qui comprend ces deux composés présente un effet bactéricide synergique sur l'activité de la flore intestinale. En effet, le composé de méthionine biodisponible et l'huile essentielle, ou le mélange d'huiles essentielles, renforcent mutuellement leurs effets.

Aucun document de l'art antérieur ne décrit l'effet bactéricide synergique d'un composé de méthionine biodisponible et d'au moins une huile essentielle.

Par "méthionine biodisponible", on entend tout composé susceptible de complémenter les besoins journaliers des animaux d'élevage en méthionine. En effet, la méthionine peut être administrée chez les animaux d'élevage sous différentes formes.

Selon la présente invention, le composé de méthionine biodisponible se trouve à l'état isolé.

Il peut s'agir tout d'abord de la méthionine elle-même, notamment de L-méthionine ou de D,L-méthionine.

Il peut s'agir également d'un dérivé de la méthionine. Par "dérivé de la méthionine", on entend par exemple les sels, les amides, les esters alkyliques et alcooliques, les dérivés cétoniques et les hydroxy-analogues de la méthionine et leurs dérivés eux-mêmes.

Il peut s'agir de l'acide 2-hydroxy-4-méthylthiobutanoïque (ci-après appelé HMTBA ou hydroxy-analogue de méthionine), connu comme analogue de la méthionine pour nourrir les animaux d'élevage. Il a l'avantage de se présenter sous forme liquide, ce qui facilite son utilisation par les sociétés productrices d'aliments.

Il peut également s'agir de l'ester isopropylique de l'hydroxy-analogue de la méthionine, ou de l'ester tertio-butylique

de la méthionine.
La composition alimentaire de l'invention peut également comprendre plusieurs sources de méthionine biodisponible, par exemple un mélange de méthionine et de son hydroxy-analogue.

La biodisponibilité de la source de méthionine passe par la détermination du taux en composé actif dans le sang, par rapport à la quantité de composé actif introduite dans la ration de l'animal. Cette détermination prend en compte le taux d'absorption dans l'intestin au cours du transit digestif, le passage du bol alimentaire dans les différents estomacs des animaux polygastriques et le taux de transformation du composé actif par l'organisme (c'est par exemple le cas de l'hydroxy-analogue de méthionine).

Par "huile essentielle", on entend un liquide obtenu à partir de plantes (fleurs, bourgeons, graines, feuilles, brindilles, herbes, écorces, bois, fruits et racines). L'huile essentielle peut notamment être obtenue par expression, fermentation, enfleurage ou extraction. Les huiles essentielles sont parfois appelées huiles éthérées ou volatiles, du fait de la présence de terpènes (hydrates de carbone non aromatiques) et de composés oxygénés (alcools, aldéhydes, cétones) qui sont aromatiques. L'huile essentielle peut être brute, dépenténée, rectifiée ou composée.

L'huile essentielle peut, selon la présente invention, être un mélange d'huiles essentielles.

Selon un mode de réalisation de la présente invention, les huiles essentielles sont composées là plus de 5% de l'un au moins des composés choisis parmi l'acétate de linalyle, l'alcool cuminique, l'aldéhyde cinnamique, le bornéol, le cadinène, le camphène, le camphre, le carvacrol, la carvone, le cinéol, le citral, le citronellal, le citronellol, le cymène, le dipentène, l'estragol, l'eugénol, le géraniol, le limonène (D ou L), le linalol (D ou L), le menthol, le méthylchavicol, le paracymène, le phéllandrène, le pinène (alpha ou bêta), le salicylate de méthyle, le terpinène (alpha et bêta), le terpinéol (alpha et bêta), la thuyone, le thymol, l'acétate de bornyle, l'acétate de géranyle, l'acétate d'eugényle, l'aldéhyde cuminique, l'allicine, l'anethol, le périllaldéhyde et le sabinène. Elles sont préférentiellement composées à plus de 10% de l'un au moins de ces composés.

Les huiles utilisées sont préférentiellement extraites par expression à froid des zestes ou par distillation de diverses parties des végétaux suivants : ail, airelle, aloé, aneth, anis vert, arbre à thé, basilic, bergamote, bois de rose, bouleau, cade, carotte, curcuma, cajeput, camphre, cannelle, carvi, céleri, chêne, citron, citronnelle, coriandre, cumin, estragon, eucalyptus globuleux, fenouil, genévrier, géranium bourbon, gingembre, ginseng, girofle, hysope, laurier noble, lavande vraie, lavande aspic, lemongrass, limette, mandarine, marjolaine, menthe poivrée, muscade, myrrhe, myrte, niaouli, oignon, oliban, orange, origan, palmarosa, pamplemousse, papaye, paprika, patchouli, persil, piment, pin maritime, pin sylvestre, pomme, raifort, romarin, santal, sarriette, sassafras, sauge, serpolet, térébenthine, thym rouge, verveine, vétiver, ylang-ylang.

Par "animal", on entend plus particulièrement les animaux d'élevage et notamment les animaux de pacage (notamment les bovins élevés pour la viande, le lait, le fromage et le cuir ; les ovins élevés pour la viande, la laine et le fromage ; les caprins), les porcins, les lapins, les volailles (les poulets, les poules, les dindes, les canards, les oies et autres), les animaux aquatiques (par exemple les poissons, les crevettes, les huîtres et les moules), les animaux de loisir et de compagnie (notamment le cheval, le chien, le chat ). Les bovins, ou bovinés, constituent une sous-famille des bovidés, mammifères ruminants polygastriques, qui comprend plusieurs espèces importantes d'animaux d'élevage (races laitières, races à viande et races mixtes).

La composition de l'invention se trouve sous forme de poudre ou sous forme liquide. La méthionine biodisponible, quant à elle, se trouve sous forme de poudre ou de granulés. Ceci implique, en outre, la dilution de cette poudre dans l'huile essentielle afin de constituer la composition selon la présente invention. Comme décrit plus haut, la méthionine peut également se trouver sous forme d'hydroxy-analogue, donc sous forme liquide. Dans ce cas, les deux liquides sont mélangés de façon homogène avant d'être administrés aux animaux ou mélangés à la ration de l'animal.

De préférence, le composé a) est choisi parmi la méthionine elle-même (L- méthionine ou D,L-méthionine), ou un de ses dérivés, de type sel, amide, ester alkylique et alcoolique, dérivé cétonique, hydroxy-analogue ou un dérivé de ces produits.

Selon un mode de réalisation de la présente invention, le composé a) a pour formule générale (I) :

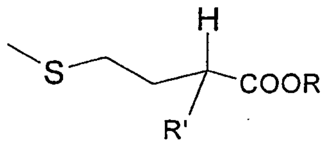

dans laquelle

R représente H ou un groupement isopropyle et
R' représente -OH ou -NH2.

Selon un mode de réalisation de la présente invention, la composition alimentaire de la présente invention qui présente un effet bactéricide synergique sur la flore intestinale des animaux, comprend :

a) de 5 à 95% en poids d'un composé de méthionine biodisponible,
b) de 5 à 95% en poids d'au moins une huile essentielle, et
c) éventuellement au moins un autre composé.

Selon ce mode de réalisation, la composition alimentaire est susceptible de comprendre au moins un autre composé, par exemple un émulsifiant ou de la gélatine. Il s'agit selon ce mode de réalisation d'une composition ouverte.
Avantageusement, ladite composition comprend de 10 à 90% en poids d'un composé de méthionine biodisponible, de 10 à 90% en poids d'au moins une huile essentielle, et éventuellement au moins un autre composé.
Selon un autre mode encore de réalisation de la présente invention, ladite composition comprend de 15 à 85% en poids d'un composé de méthionine biodisponible, de 15 à 85% en poids d'au moins une huile essentielle, et éventuellement au moins un autre composé.
Selon un autre mode de réalisation de la présente invention, ladite composition comprend de 20 à 80% en poids d'un composé de méthionine biodisponible, de 20 à 80% en poids d'au moins une huile essentielle, et éventuellement au moins un autre composé.
Selon un tout autre mode de réalisation de la présente invention, la composition alimentaire et qui présente un effet bactéricide synergique sur la flore intestinale des animaux, consiste en :

a) 5 à 95% en poids d'un composé de méthionine biodisponible, et
b) 5 à 95% en poids d'au moins une huile essentielle.

Selon ce mode de réalisation, la composition alimentaire ne comprend que ces deux composés, elle n'en contient aucun autre. Il s'agit selon ce mode de réalisation d'une composition fermée.
Avantageusement, la composition alimentaire de la présente invention consiste en 10 à 90% en poids d'un composé de méthionine biodisponible, et en 10 à 90% en poids d'au moins une huile essentielle.
Selon un autre mode encore de réalisation de la présente invention, ladite composition consiste en 15 à 85% en poids d'un composé de méthionine biodisponible et en 15 à 85% en poids d'au moins une huile essentielle.
Selon un autre mode de réalisation de la présente invention, ladite composition comprend de 20 à 80% en poids d'un composé de méthionine biodisponible, de 20 à 80% en poids d'au moins une huile essentielle, et éventuellement au moins un autre composé.
Selon un mode de réalisation de la présente invention, la méthionine biodisponible (i.e. composé a)) est présente au sein de la composition en une proportion supérieure à 60% en poids, de préférence supérieure à 80% en poids.
Selon un mode de réalisation de la présente invention, l'huile essentielle (i.e. le composé b)) est présente au sein de la composition en une proportion inférieure à 40% en poids, de préférence inférieure à 20% en poids, de manière encore plus préférée inférieure à 10% en poids. De même, selon un mode de réalisation de la présente invention, le composé b) est présent au sein de la composition en une proportion supérieure à 5% en poids, de préférence supérieure ou égale à 8% en poids.
La présente invention concerne également un additif alimentaire comprenant la composition selon la présente invention.
Par "additif alimentaire", on entend un composé actif ou un mélange de composés actifs entrants dans la composition de l'aliment dans une proportion généralement inférieure à 2%, par exemple inférieure à 1 % en poids de l'aliment.
La présente invention concerne également un aliment pour animal, notamment du type ration alimentaire, comprenant la composition alimentaire telle que ci-dessus définie ou comprenant ledit additif alimentaire.
La présente invention concerne enfin l'utilisation de la composition telle que ci-dessus définie, pour obtenir un additif alimentaire.
Selon un mode de réalisation de la présente invention, une telle utilisation peut en outre permettre d'abaisser l'activité de la flore endogène chez les animaux d'élevage ou permettre de combattre les effets néfastes des microorganismes pathogènes de la flore intestinale des animaux d'élevage.
Selon un mode de réalisation de la présente invention, une telle utilisation peut en outre permettre d'améliorer les performances des animaux d'élevage telles que la croissance, la viabilité, l'homogénéité, l'efficacité de production.
Selon un autre mode de réalisation de la présente invention, la composition améliore les performances par une réduction de l'activité de la flore intestinale des animaux d'élevage.
Ainsi selon la présente invention, on utilise la composition de la présente invention pour préparer un additif alimentaire pour animaux d'élevage pour améliorer les performances par une réduction de l'activité de la flore intestinale desdits animaux.
Les exemples ci-dessous et les figures permettront de mettre en évidence certains avantages et caractéristiques

de la présente invention.

L'objectif des essais qui suivent est de comparer la production de gaz par la flore du contenu iléal de poulets de chair, sous l'effet de différents items d'essai. La production de gaz est un indicateur indirect de l'activité bactérienne de la flore iléale du poulet.

Exemple 1

*Animaux et prélèvements*

50 poulets de chair sont élevés avec un régime à base de blé. Ces poulets sont élevés avec le même régime alimentaire. Les poulets sont sacrifiés au 35$^{ème}$ jour, puis on prélève le contenu de leur iléon dans des conditions anaérobies. Le contenu est placé dans des flacons qui sont conservés à -18°C.

*Tests in vitro*

A partir du schéma expérimental décrit dans le tableau I, ci-dessous, on mesure le volume de gaz produit en fonction du temps.

48 flacons sont remplis successivement par :

- le traitement (rien pour le témoin négatif, le HMTBA, l'HE ou l'item d'essai),
- 90 ml de solution tampon, contenant de l'amidon de blé (milieu nutritif pour les bactéries) ; cette solution tampon a pour fonction de maintenir le pH à valeur constante et de supprimer l'$O_2$
- 10 ml d'inoculum, constitué d'un mélange de ¼ de contenu iléal intestinal et de ¾ de solution physiologique stérile, qui est filtré et homogénéisé avant le remplissage.

Les doses testées sont calculées pour être représentatives des concentrations des produits que l'on retrouverait dans le contenu gastro-intestinal des animaux.

| Traitement | | Témoin négatif | HMTBA | HE 1 | Item d'essai 1 | HE 2 | Item d'essai 2 | HE 3 | Item d'essai 3 | HE 4 | Item d'essai 4 | HE 5 | Item d'essai 5 |
|---|---|---|---|---|---|---|---|---|---|---|---|---|---|
| item | | 0 | HMTBA | HE 1 | HMTBA + HE 1 | HE 2 | HMTBA + HE 2 | HE 3 | HMTBA + HE 3 | HE 4 | HMTBA + HE 4 | HE 5 | HMTBA + HE 5 |
| répétitions | | 4 | 4 | 4 | 4 | 4 | 4 | 4 | 4 | 4 | 4 | 4 | 4 |
| dose d'HMTBA apporté | mg | 0 | 83 | 0 | 83 | 0 | 83 | 0 | 83 | 0 | 83 | 0 | 83 |
| | % en poids | 0 | 100 | 0 | 75 | 0 | 62,5 | 0 | 71,4 | 0 | 73 | 0 | 62,5 |
| dose de HE apportée | mg | 0 | 0 | 28 | 28 | 50 | 50 | 34 | 34 | 31 | 31 | 50 | 50 |
| | % en poids | 0 | 0 | 100 | 25 | 100 | 37,5 | 100 | 28,6 | 100 | 27 | 100 | 37,5 |
| inoculum (ml) | | 10 | 10 | 10 | 10 | 10 | 10 | 10 | 10 | 10 | 10 | 10 | 10 |
| solution tampon (ml) | | 90 | 90 | 90 | 90 | 90 | 90 | 90 | 90 | 90 | 90 | 90 | 90 |

HE : Huile Essentielle ou mélange d'huiles essentielles
HMTBA : méthionine biodisponible sous forme d'acide 2-hydroxy-4-méthylthiobutanoïque

Tableau I

A partir du schéma expérimental décrit dans le tableau I, ci-dessus, on obtient des courbes de volume de gaz produit en fonction du temps, qui ont pour la plupart une forme sigmoïde.

Ces courbes sont modélisées selon le modèle suivant :

$$Y = A / (1 + (C/t) ^ B)$$

où Y = volume de gaz produit
t = temps
A, B et C = paramètres du modèle

A partir de ces équations, on analyse alors le volume final modélisé Vf (Vf=A), ainsi que le temps pour lequel le taux de production de gaz est maximal, appelé Trm (ce temps est utilisé comme une mesure indirecte du temps de latence, qui est inaccessible avec ce modèle). On réalise une analyse de variance (ANOVA) sur ces données pour évaluer l'effet de l'item. Si la probabilité de l'effet traitement est significative, c'est-à-dire si $p<0,05$, les moyennes sont alors comparées par le test de PLSD de Fisher qui permet d'établir les différences sur les moyennes entre les différents traitements.

*Résultats*

Les résultats apparaissent dans le tableau II, ci-dessous.
Dans le tableau II, les valeurs des paramètres sont significativement différentes au seuil de 5% lorsque les traitements n'ont aucune lettre en commun.

Dans un premier temps, on observe que toutes les compositions testées donnent des résultats significativement différents du témoin sur les deux critères, à savoir le Vf et le Trm. Ces compositions ont donc un effet réducteur sur l'activité de la flore iléale.

L'objectif est de maximiser le Trm. Tous les items d'essai sont donc efficaces.

On démontre qu'il y a un effet synergique entre le composé de méthionine biodisponible, en l'espèce l'HMTBA, et l'huile essentielle sur l'activité bactérienne.

En effet, le Trm du témoin négatif est de 10,8h. Le traitement au HMTBA donne un Trm identique, ce qui laisse sous-entendre que le HMTBA n'a pas ou peu d'effet sur le démarrage de l'activité bactérienne lorsqu'il est apporté seul. Le traitement HE1 donne un Trm de 13,5h. HE1 a donc un effet sur l'activité bactérienne.
Le mélange 1 quant à lui donne un Trm de 19,3h. Le mélange 1 présente donc un effet sur l'activité bactérienne qui va au-delà de la somme des effets de HE1 et de HMTBA isolément.
Le mélange 1, ainsi que les mélanges 2 à 5 qui donnent le même type de résultats, présentent donc un effet synergique sur l'activité bactérienne mesurée par le Trm.

La mesure du Vf est un indicateur de l'effet à long terme du traitement. L'objectif est de minimiser le Vf.

Les items considérés comme les plus efficaces sont ceux qui minimisent le volume final et, dans le même temps, maximisent le Trm.
Il s'agit des mélanges 3 et 5.

| Traitement | Vf (ml) Moy | Vf (ml) Ecart-type | Vf (ml) différence | Trm (h) Moy | Trm (h) Ecart-type | Trm (h) différence |
|---|---|---|---|---|---|---|
| Témoin négatif | 586 | 17 | a | 10,8 | 0,2 | a |
| HMTBA | 565 | 21 | a | 10,8 | 0,2 | a |
| HE 1 | 496 | 41 | b | 13,5 | 0,1 | b |
| Item d'essai 1 | 469 | 18 | bc | 19,3 | 1,8 | c |
| HE 2 | 238 | 34 | ef | 28,6 | 1,2 | d |
| Item d'essai 2 | 184 | 17 | f | 46,3 | 3,1 | f |
| HE 3 | 460 | 9 | c | 21,5 | 4,6 | c |
| Item d'essai 3 | 237 | 48 | ef | 39,1 | 3,4 | e |
| HE 4 | 302 | 113 | d | 28,9 | 0,4 | d |
| Item d'essai 4 | 252 | 18 | de | 35,8 | 1,3 | e |
| HE 5 | 308 | 55 | d | 30,8 | 1,9 | d |
| Item d'essai 5 | 193 | 8 | f | 37,2 | 3,1 | e |

Vf : volume final de gaz produit (ml)

Trm : temps pour lequel le taux de production de gaz est maximal (heure).

Item d'essai n = HE n + HMTBA

Tableau II

Exemple 2

Les conditions et paramètres d'expérimentation de l'exemple 1 sont repris à l'exemple 2, selon le tableau III ci-dessous.

| Traitement | | Témoin négatif | HMTBA | HE 1 | Item d'essai 1 | HE 2 | Item d'essai 2 |
|---|---|---|---|---|---|---|---|
| item | | 0 | HMTBA | HE 1 | HMTBA + HE 1 | HE 2 | HMTBA + HE 2 |
| répétitions | | 4 | 4 | 4 | 4 | 4 | 4 |
| dose d'HMTBA apporté | mg | 0 | 83 | 0 | 83 | 0 | 83 |
| | % en poids | 0 | 100 | 0 | 84,7 | 0 | 84,7 |
| dose de HE apportée | mg | 0 | 0 | 15 | 15 | 15 | 15 |
| | % en poids | 0 | 0 | 100 | 15,3 | 100 | 15,3 |
| inoculum (ml) | | 10 | 10 | 10 | 10 | 10 | 10 |
| solution tampon (ml) | | 90 | 90 | 90 | 90 | 90 | 90 |

HE1 : thym rouge

HE2 : arbre à thé

HMTBA : méthionine biodisponible sous forme d'acide 2-hydroxy-4-méthylthiobutanoïque

Tableau III

*Résultats*

Les résultats apparaissent dans le tableau IV ci-dessous.

| Traitement | | Témoin négatif | HMTBA | HE 1 | Item d'essai 1 | HE 2 | Item d'essai 2 |
|---|---|---|---|---|---|---|---|
| Vf (ml) | Moy | 542,0 | 296,2 | 226,7 | 203,1 | 319,6 | 361,1 |
| | Ecart-type | 43,9 | 28,6 | 15,5 | 15,6 | 10,8 | 41,3 |
| | différence | **a** | **b** | **b** | **b** | **b** | **e** |
| Trm (h) | Moy | 15,3 | 20,3 | 27,7 | 33,1 | 20,2 | 24,8 |
| | Ecart-type | 1,0 | 0,7 | 1,1 | 0,8 | 0,8 | 0,8 |
| | différence | **a** | **b** | **c** | **d** | **b** | **e** |

Vf : volume final de gaz produit (ml)

Trm : temps pour lequel le taux de production de gaz est maximal (heure).

Item d'essai n = HE n + HMTBA

Tableau IV

Concernant le Trm qui doit être maximisé, tous les traitements testés (HMTBA, HE et item d'essai) sont donc efficaces.

On démontre à nouveau qu'il y a un effet synergique entre le composé de méthionine biodisponible, en l'espèce l'HMTBA, et l'huile essentielle sur l'activité bactérienne.
En effet, le Trm du témoin négatif est de 15,3h.
Le traitement HMTBA donne un Trm de 20,3h. Ainsi, le HMTBA a donc un effet sur l'activité bactérienne lorsqu'il est apporté seul.
Le traitement HE1 donne un Trm de 27,7h. HE1 a donc un effet sur l'activité bactérienne.
Le mélange 1 (ou item d'essai 1) quant à lui donne un Trm de 33,1 h. Le mélange 1 présente donc un effet sur l'activité bactérienne qui va au-delà de la somme des effets de HE1 et de HMTBA isolément.

Le mélange 1, ainsi que le mélange 2 qui donne le même type de résultats, présentent donc un effet synergique.

La mesure du Vf est un indicateur de l'effet à long terme du traitement. L'objectif est de minimiser le Vf.

Les items considérés comme les plus efficaces sont ceux qui minimisent le volume final et, dans le même temps, maximisent le Trm.
Il s'agit du mélange 1.

NB : Les différences dans les valeurs de Vf et Trm entre les différents exemples de la présente demande peuvent être dues à l'emploi de différents contenus digestifs, issus de différents lots d'animaux. Bien que l'élevage, l'alimentation et les conditions de prélèvement soient identiques, les populations bactériennes qui s'implantent au niveau digestif en élevage ne sont jamais rigoureusement les mêmes. Les variations de valeur observées en fonction des exemples sont dues à la variation du type de flore présente dans les différents prélèvements.

Exemple 3

Les conditions et paramètres d'expérimentation de l'exemple 1 sont repris à l'exemple 3, selon le tableau V ci-dessous.

| Traitement | | Témoin négatif | HMTBA | HE 1 | Item d'essai 1 |
|---|---|---|---|---|---|
| item | | 0 | HMTBA | HE 1 | HMTBA + HE1 |
| répétitions | | 4 | 4 | 4 | 4 |
| dose d'HMTBA apporté | mg | 0 | 83 | 0 | 83 |
| | % en poids | 0 | 100 | 0 | 84,7 |
| dose de HE apportée | mg | 0 | 0 | 15 | 15 |
| | % en poids | 0 | 0 | 100 | 15,3 |
| inoculum (ml) | | 10 | 10 | 10 | 10 |
| solution tampon (ml) | | 90 | 90 | 90 | 90 |

HE1 : cannelle

HMTBA : méthionine biodisponible sous forme d'acide 2-hydroxy-4-méthylthiobutanoïque

Tableau V

*Résultats*

Les résultats apparaissent dans le tableau VI ci-dessous.

| Traitement | | Témoin négatif | HMTBA | HE 1 | Item d'essai 1 |
|---|---|---|---|---|---|
| Vf (ml) | Moy | 632,7 | 334,3 | 218,3 | 20,0 |
| | Ecart-type | 23 | 86 | 82 | 10 |
| | différence | **a** | **b** | **c** | **e** |
| Trm (h) | Moy | 14,1 | 28,1 | 53,5 | > 72 |
| | Ecart-type | 0,1 | 2,2 | 10,9 | nd |
| | différence | **a** | **b** | **c** | **d** |

Vf : volume final de gaz produit (ml)

Trm : temps pour lequel le taux de production de gaz est maximal (heure).

Item d'essai n = HE n + HMTBA

nd : non déterminé

Tableau VI

Toutes les compositions testées donnent des résultats significativement différents du témoin sur les deux critères, à savoir le Vf et le Trm. Ces compositions ont donc un effet réducteur sur l'activité de la flore iléale.

Concernant le Trm qui doit être maximisé, tous les traitements testés (HMTBA, HE et item d'essai) sont donc efficaces.

On démontre à nouveau qu'il y a un effet synergique entre le composé de méthionine biodisponible, en l'espèce l'HMTBA, et l'huile essentielle, en l'espèce l'huile essentielle de cannelle, sur l'activité bactérienne.
En effet, le Trm du témoin négatif est de 14,1h.
Le traitement HMTBA donne un Trm de 28,1 h. Ainsi, le HMTBA a un effet sur l'activité bactérienne lorsqu'il est apporté seul.
Le traitement HE1 donne un Trm de 53,5h. HE1 a donc un effet sur l'activité bactérienne.

Le mélange 1 (ou item d'essai 1) quant à lui donne un Trm "supérieur à 72h". Il présente donc une activité bactéricide quasi-totale qui a empêché la production de gaz par les bactéries issues du contenu digestif. La combinaison d'HMTBA et d'huile essentielle de cannelle présente donc un effet très fort sur l'activité bactérienne, effet qui va au-delà de la somme des effets de l'huile essentielle de cannelle et du HMTBA isolément.
Le mélange 1 présente donc un fort effet synergique. Voir la Figure 6.

La mesure du Vf est un indicateur de l'effet à long terme du traitement. L'objectif est de minimiser le Vf.

Les items considérés comme les plus efficaces sont ceux qui minimisent le volume final et, dans le même temps, maximisent le Trm.
Le mélange 1 répond à ces caractéristiques et il peut donc être considéré comme particulièrement efficace.

Exemple 4

Les conditions et paramètres d'expérimentation de l'exemple 1 sont repris à l'exemple 4, selon le tableau VII ci-dessous.

Tableau VII

| Traitement | | Témoin négatif | HMTBA | MHE | Item d'essai |
|---|---|---|---|---|---|
| item | | 0 | HMTBA | MHE2 | HMTBA + MHE2 |
| répétitions | | 4 | 4 | 4 | 4 |
| dose d'HMTBA apporté | mg | 0 | 83 | 0 | 83 |
| | % en poids | 0 | 100 | 0 | 84,7 |
| dose de HE apportée | mg | 0 | 0 | 15 | 15 |
| | % en poids | 0 | 0 | 100 | 15,3 |
| inoculum (ml) | | 10 | 10 | 10 | 10 |
| solution tampon (ml) | | 90 | 90 | 90 | 90 |
| MHE : $^{3}/_{4}$ arbre à thé $^{1}/_{4}$ huile essentielle de cannelle<br>HMTBA : méthionine biodisponible sous forme d'acide 2-hydroxy-4-méthylthiobutanoïque | | | | | |

*Résultats*

Les résultats apparaissent dans le tableau VIII ci-dessous.

Tableau VIII

| Traitement | | Témoin négatif | HMTBA | MHE | Item d'essai |
|---|---|---|---|---|---|
| Vf(ml) | Moy | 518 | 421 | 377 | 253 |
| | Ecart-type | 35 | 37 | 21 | 2 |
| | différence | **a** | **b** | **b** | **d** |
| Tm(h) | Moy | 11,1 | 15,4 | 19,0 | 25,3 |
| | Ecart-type | 1,4 | 1,7 | 2,7 | 2,6 |
| | différence | **a** | **b** | **d** | **e** |
| Vf : volume final de gaz produit (ml)<br>Tm : temps pour lequel le taux de production de gaz est maximal (heure). | | | | | |

On démontre à nouveau qu'il y a un effet synergique entre le composé de méthionine biodisponible et les mélanges d'huile essentielle sur l'activité bactérienne.

Le Trm du témoin négatif est de 11,1 h.

Concernant l'item d'essai qui consiste en 84,7% d'HMTBA et 15,3% d'un mélange ¾ d'huile essentielle d'arbre à thé et de ¼ d'huile essentielle de cannelle :

- le traitement HMTBA donne un Trm de 15,4h. Ainsi, le HMTBA a un effet sur l'activité bactérienne lorsqu'il est apporté seul (différence avec le témoin négatif), valeur de la différence : 4,3h
- le traitement MHE donne un Trm de 19,0h. HE1 a donc un effet sur l'activité bactérienne (différence avec le témoin négatif), valeur de la différence : 7,9h
- Le mélange (ou item d'essai) quant à lui donne un Trm de 25,3h. Le mélange présente donc un effet sur l'activité bactérienne qui va au-delà de la somme des effets de MHE2 et de HMTBA isolément. En effet, (25,3-11,1) est supérieure à (4,3+7,9).
  Le mélange présente donc un effet synergique sur l'activité bactérienne.

La mesure du Vf est un indicateur de l'effet à long terme du traitement. L'objectif est de minimiser le Vf.

Les items considérés comme les plus efficaces sont ceux qui minimisent le volume final et, dans le même temps, maximisent le Trm.

Le mélange de cet essai répond à ces caractéristiques et peut donc être considéré comme particulièrement efficace.

Exemple 5

On a montré avec l'exemple 1 l'efficacité des 5 mélanges d'huiles essentielles pour réduire l'activité de la flore iléale issue d'animaux nourris avec un régime à base de blé.
L'objectif de cet essai est de démontrer que trois de ces mélanges ont également un effet réducteur sur l'activité de la flore iléale issue de poulets nourris avec un régime à base de maïs.

*Animaux et prélèvements*

Conditions identiques à celles de l'exemple 1.

*Tests in vitro*

Le schéma expérimental est présenté dans le Tableau I pour les mélanges 2, 3, 4 et 5.

*Résultats*

Les résultats apparaissent dans le Tableau IX ci-après.

| Traitement | | Témoin négatif | HE2 + HMTBA | HE 3 + HMTBA | HE4 + HMTBA | HE5 + HMTBA |
|---|---|---|---|---|---|---|
| Vf (ml) | Moy | 455 | 203 | 201 | 228 | 206 |
| | Ecart-type | 11,8 | 8,4 | 1,5 | 14,4 | 13,9 |
| | différence | **a** | **b** | **b** | **b** | **b** |
| Trm (h) | Moy | 17,1 | 30,1 | 22,1 | 28,0 | 30,3 |
| | Ecart-type | 0,68 | 1,86 | 1,18 | 0,63 | 2,20 |
| | différence | **a** | **d** | **b** | **c** | **d** |

HE : Huile Essentielle ou mélange d'huiles essentielles

Tableau IX

Dans le tableau, les valeurs des paramètres sont significativement différentes au seuil de 5% lorsque les traitements n'ont aucune lettre en commun.
Les 4 mélanges testés diminuent significativement le Vf et augmentent significativement le Trm par rapport au témoin. Par conséquent, ces produits sont efficaces pour réduire l'activité de la flore iléale de poulet nourris avec un régime à base de maïs, aussi bien à court terme qu'à plus long terme, comme c'était le cas avec la flore de poulet nourris avec un régime à base de blé (exemple 1).
Ces mélanges sont donc efficaces avec les deux types de régimes utilisés.

Exemple 6

On a montré avec les exemples 1 et 5 l'efficacité de plusieurs mélanges d'huiles essentielles pour réduire l'activité de la flore iléale issue de poulets de chair.
L'objectif de cet essai est de connaître l'efficacité de ces mélanges sur de la flore caecale de porc.

*Animaux*

Des porcs sont élevés jusqu'au 100$^{ème}$ jour, puis on prélève le contenu de leur caecum dans des conditions anaérobies.

*Tests in vitro*

Le schéma expérimental est présenté dans le Tableau I pour les mélanges 2, 3 et 4.

*Résulta ts*

Les résultats apparaissent dans le Tableau X.
Dans le tableau, les valeurs des paramètres sont significativement différentes au seuil de 5% lorsque les traitements n'ont aucune lettre en commun.

| Traitement | | Témoin négatif | HE 2 + HMTBA | HE3 + HMTBA | HE4 + HMTBA |
|---|---|---|---|---|---|
| Vf (ml) | Moy | 430 | 265 | 344 | 412 |
| | Ecart-type | 48,0 | 2,5 | 27,4 | 17,4 |
| | différence | **ab** | **d** | **c** | **b** |
| Trm (h) | Moy | 21,1 | 23,9 | 25,8 | 25,2 |
| | Ecart-type | 0,36 | 1,25 | 2,04 | 1,30 |
| | différence | **a** | **bc** | **c** | **c** |

HE : Huile Essentielle ou mélange d'huiles essentielles

Tableau X

Tous les mélanges testés diminuent le Vf, significativement pour 2 et 3, en moyenne pour 4, et augmentent significativement le Trm par rapport au témoin. Les mélanges sont donc efficaces pour réduire l'activité de la flore caecale de porc, aussi bien à court terme qu'à plus long terme.
Ainsi 3 mélanges fonctionnent à la fois sur la flore de poulet et de porc.

Exemple 7

L'objectif de cet essai est de comparer l'efficacité d'au moins une huile essentielle et du HMTBA lorsque ces composés sont apportés séparément dans les bouteilles (les dieux produits se retrouvent en faible quantité dans 100ml de solution ; il y a donc peu de contact direct entre les molécules), ou après avoir été mélangés préalablement à leur ajout à la solution.

*Animaux*

On utilise pour cet essai de la flore iléale issue de poulet de 35 jours nourris avec un régime à base de blé.

*Tests in vitro*

On teste dans cet essai un des mélanges testés dans l'exemple 1. Le schéma expérimental est présenté dans le Tableau I pour le mélange 2.

*Résultats*

Les résultats apparaissent dans le Tableau XI.

Dans le tableau, les valeurs des paramètres sont significativement différentes au seuil de 5% lorsque les traitements n'ont aucune lettre en commun.

Tableau XI

| Traitement | | **Témoin négatif** | **HE 2 + HMTBA** Après mélange | **HE 2 + HMTBA** Apport séparé |
|---|---|---|---|---|
| Vf(ml) | Moy | 532 | 184 | 407 |
| | Ecart-type | 20,4 | 23,3 | 24,4 |
| | différence | **a** | **c** | **b** |
| Tm(h) | Moy | 14,8 | 31,3 | 19,4 |
| | Ecart-type | 0,45 | 3,31 | 0,66 |
| | différence | **a** | **c** | **a** |
| HE : Huile Essentielle ou mélange d'huiles essentielles | | | | |

L'effet de HE2 + HMTBA est très supérieur lorsque les deux composés sont mélangés avant l'introduction dans la bouteille : le contact préalable améliore particulièrement l'action des composés sur la flore. On peut donc penser qu'il y a des interactions physico-chimiques entre les composés, ces interactions modifiant leur efficacité. Ceci confirme l'effet synergique du mélange d'un composé de méthionine biodisponible avec au moins une huile essentielle.

Exemple 8

*Animaux*

On utilise pour cet essai de la flore iléale issue de poulet de 35 jours nourris avec un régime à base de blé.

*Tests in vitro*

Le schéma expérimental est présenté dans le Tableau XII ci-dessous.

Tableau XII

| **Traitement** | **Témoin négatif** | **HMTBA** | **MHE** | **Item d'essai** |
|---|---|---|---|---|
| **item** | 0 | HMTBA | HE | HMTBA + HE |
| **répétitions** | 4 | 3 | 3 | 3 |

(suite)

| Traitement | | Témoin négatif | HMTBA | MHE | Item d'essai |
|---|---|---|---|---|---|
| dose d'HMTBA apporté | mg | 0 | 113 | 0 | 113 |
| | % en poids | 0 | 100 | 20 | 89,7 |
| dose de HE apportée | mg | 0 | 0 | 13 | 13 |
| | % en poids | 0 | 0 | 100 | 10,3 |
| inoculum (ml) | | 10 | 10 | 10 | 10 |
| solution tampon (ml) | | 90 | 90 | 90 | 90 |
| HE : huile d'origan | | | | | |

*Résultats*

Les résultats apparaissent dans le Tableau XIII ci-dessous. Dans le tableau, les valeurs des paramètres sont significativement différentes au seuil de 5% lorsque les traitements n'ont aucune lettre en commun.

Tableau XIII

| Traitement | | Témoin négatif | HMTBA | HE | HE + HMTBA |
|---|---|---|---|---|---|
| Vf(ml) | Moy | 407 | 353 | 300 | 224 |
| | Ecart-type | 45,1 | 17,7 | 127,6 | 1,9 |
| | différence | **a** | **a** | **a** | **a** |
| Tm(h) | Moy | 16,0 | 16,2 | 18,3 | 20,3 |
| | Ecart-type | 1,67 | 0,71 | 0,99 | 0,67 |
| | différence | **a** | **a** | **a** | **a** |

Lorsqu'il est utilisé seul, le HMTBA n'a pas d'effet significatif sur la fermentation, comme observé dans l'exemple 1.
L'huile d'origan augmente le Trm de 2,3 heures en moyenne par rapport au témoin négatif (effet significatif).
Le mélange de l'huile d'origan et du HMTBA augmente le Trm de 4,3 heures par rapport au témoin négatif. L'effet du mélange est synergique sur l'activité de la flore bactérienne. L'huile et le HMTBA agissent donc en synergie pour réduire à court terme l'activité de la flore.

## Revendications

**1.** Composition synergique de méthionine biodisponible destinée à l'alimentation des animaux d'élevage, comprenant :

a) un composé de méthionine biodisponible, et
b) au moins 5% en poids d'au moins une huile essentielle.

**2.** Composition selon la revendication 1, comprenant :

a) de 5 à 95% en poids d'un composé de méthionine biodisponible,
b) de 5 à 95% en poids d'au moins une huile essentielle, et
c) éventuellement au moins un autre composé.

**3.** Composition selon la revendication 1, consistant en :

a) de 5 à 95% en poids d'un composé de méthionine biodisponible, et
b) de 5 à 95% en poids d'au moins une huile essentielle.

4. Composition selon l'une quelconque des revendications précédentes, dans laquelle le composé a) est choisi parmi la méthionine elle-même (L- méthionine ou D,L-méthionine), ou un de ses dérivés, de type sel, amide, ester alkylique et alcoolique, dérivé cétonique, hydroxy-analogue ou un dérivé de ces produits.

5. Composition selon l'une quelconque des revendications précédentes, dans laquelle le composé a) a pour formule générale (I) :

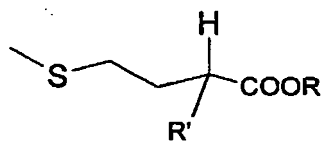

dans laquelle

R représente H ou un groupement isopropyle et
R' représente -OH ou $-NH_2$.

6. Utilisation de la composition selon l'une quelconque des revendications 1 à 5 pour obtenir un aliment destiné à l'alimentation animale.

7. Utilisation de la composition selon l'une quelconque des revendications 1 à 5, pour obtenir un additif alimentaire.

8. Utilisation selon la revendication 7, pour obtenir un additif alimentaire pour abaisser l'activité de la flore endogène chez les animaux d'élevage.

9. Utilisation selon la revendication 7, pour obtenir un additif alimentaire pour améliorer les performances des animaux d'élevage telles que la croissance, la viabilité, l'homogénéité, l'efficacité de production.

10. Utilisation selon la revendication 7, pour la préparation d'une solution médicamenteuse destinée à combattre les effets néfastes des microorganismes pathogènes de la flore intestinale des animaux d'élevage.

**Claims**

1. Synergistic composition of bioavailable methionine intended for the feeding of livestock, comprising:

a) a bioavailable methionine compound, and
b) at least 5 wt.% of at least one essential oil.

2. Composition according to Claim 1, comprising:

a) from 5 to 95 wt.% of a bioavailable methionine compound,
b) from 5 to 95 wt.% of at least one essential oil, and
c) optionally, at least one other compound.

3. Composition according to Claim 1, consisting of:

a) from 5 to 95 wt.% of a bioavailable methionine compound, and
b) from 5 to 95 wt.% of at least one essential oil.

4. Composition according to any one of the preceding claims, **characterized in that** compound a) is selected from methionine itself (L-methionine or D,L-methionine), or one of its derivatives, such as salt, amide, alkyl and alcoholic ester, ketone derivative, hydroxy-analogue or a derivative of these products.

5. Composition according to any one of the preceding claims, **characterized in that** compound a) is of general formula

(I):

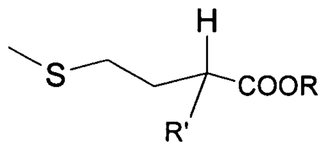

in which

R represents H or an isopropyl group and
R' represents -OH or $-NH_2$.

**6.** Use of the composition according to any one of Claims 1 to 5 to obtain a feed intended for the feeding of animals.

**7.** Use of the composition according to any one of the Claims 1 to 5, to obtain a feed additive.

**8.** Use according to Claim 7, for obtaining a feed additive for lowering the activity of the endogenous flora in livestock.

**9.** Use according to Claim 7, for obtaining a feed additive improving the performance of livestock such as growth, viability, homogeneity, and production efficiency.

**10.** Use according to Claim 7, for the preparation of a medicinal solution for combating the harmful effects of the pathogenic microorganisms of the intestinal flora of livestock.

**Patentansprüche**

**1.** Synergische Zusammensetzung von biologisch verfügbarem Methionin zur Ernährung von Zuchttieren, umfassend:

a) eine Verbindung von biologisch verfügbarem Methionin und
b) mindestens 5 Gew.-% mindestens eines essenziellen Öls.

**2.** Zusammensetzung nach Anspruch 1, umfassend:

a) 5 bis 95 Gew.-% einer Verbindung von biologisch verfügbarem Methionin,
b) 5 bis 95 Gew.-% mindestens eines essenziellen Öls und
c) gegebenenfalls mindestens eine andere Verbindung.

**3.** Zusammensetzung nach Anspruch 1, bestehend aus:

a) 5 bis 95 Gew.-% einer Verbindung von biologisch verfügbarem Methionin und
b) 5 bis 95 Gew.-% mindestens eines essenziellen Öls.

**4.** Zusammensetzung nach einem der vorhergehenden Ansprüche, wobei die Verbindung a) ausgewählt ist aus Methionin selbst (L-Methionin oder D,L-Methionin) oder einem seiner Derivate des Typs eines Salzes, Amids, Alkylesters und Alkohols, Ketonderivats, Hydroxyanalogons oder einem Derivat dieser Produkte.

**5.** Zusammensetzung nach einem der vorhergehenden Ansprüche, wobei die Verbindung a) die allgemeine Formel (I) hat:

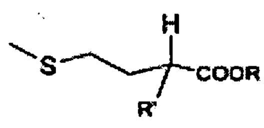

worin

R für H oder eine Isopropylgruppe steht und
R' für -OH oder $-NH_2$ steht.

6. Verwendung der Zusammensetzung nach einem der Ansprüche 1 bis 5, um ein Futtermittel für die Tierfütterung zu erhalten.

7. Verwendung der Zusammensetzung nach einem der Ansprüche 1 bis 5, um einen Futterzusatzstoff zu erhalten.

8. Verwendung nach Anspruch 7, um einen Futterzusatzstoff zur Senkung der Aktivität der endogenen Darmflora von Zuchttieren zu erhalten.

9. Verwendung nach Anspruch 7, um einen Futterzusatzstoff zur Verbesserung der Leistungen von Zuchttieren, wie Wachstum, Lebensfähigkeit, Homogenität, Produktionseffizienz, zu erhalten.

10. Verwendung nach Anspruch 7 zur Herstellung einer Arzneimittellösung für die Bekämpfung schädlicher Wirkungen von pathogenen Mikroorganismen der Darmflora von Zuchttieren.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description

- US 6322825 B
- US 4166867 A
- WO 03056935 A